# EUROPEAN PATENT APPLICATION

(11) **EP 2 579 040 A2**
(43) Date of publication of application: **10.04.2013**
(21) Application number: 11786904.0
(22) Date of filing: 25.05.2011
(51) Int. Cl.: G01N 33/573, C12Q 1/37

(54) **QUALITATIVE AND QUANTITATIVE ANALYTICAL METHOD FOR ANALYZING THE ACTIVITY TYPE OF AN ENZYME THAT IS ACTIVATED BY PROTEOLYSIS**

(30) Priority: 01.11.2010 KR 20100107640; 25.05.2010 KR 20100048665
(71) Applicant: Nanoentek, Inc., Seoul 152-724 (KR)
(72) Inventor: LEE, Chang Seop, Ansan-si Gyeonggi-do 425-080 (KR); AN, Seong Soo Alexander, Seoul 138-050 (KR); KANG, Min O, Seoul 143-202 (KR)
(74) Representative: Winkler, Andreas Fritz Ernst
(86) International application number: PCT/KR2011/003843
(87) International publication number: WO 2011/149272

(57) **Abstract**

The present invention relates to a qualitative and quantitative analytical method for analyzing the activity type of an enzyme that is activated by proteolysis. The method of the present invention is advantageous in that the activity type of an enzyme present in a specimen is analyzed using an enzyme activity inhibitor and a binder to enable the analysis to be performed in a quicker and more accurate manner as compared to enzyme-activity analysis methods using a simple binder (for example, an antibody). The method of the present invention can enable the analysis of the activity type of an enzyme present in a specimen in a simple manner using less equipment than enzyme-activity analysis methods using a simple binder. The method of the present invention involves simultaneously using the inhibitor and the binder in analyzing the activity type of a target enzyme or an enzyme to enable quick and specific analysis, and high-throughput drug screening can be performed at a high speed due to the above-described characteristics of the method of the present invention. In addition, the method of the present invention can be applied to point-of-care testing (POCT) for the clinical monitoring of the effects and dosage of a drug in a drug administration target (for example, an animal or human).

## Description

### FIELD OF THE INVENTION

The present invention relates to methods for analyzing an active form of an enzyme activated by proteolytic cleavage in a qualitative or quantitative manner.

### DESCRIPTION OF THE RELATED ART

Proteins are known to use one or more regulatory pathways for their diverse functions, To this end, various methods have been utilized in the regulator pathways, for example including cleavage, deletion or addition at a specific position in an amino acid sequence of a protein, convert or modification of its side chain, and so forth.

Of proteins, an enzyme activity has been specifically regulated through numerous mechanisms: first, a feedback regulation that the enzyme activity is regulated by a final product; second, an allosteric control including an interaction of O₂, H⁺ and CO₂ in hemoglobin or a regulation mechanism of ATCase (aspartate transcarbamoylase) activity in *E coli*; third, stimulation and inhibition by a regulatory protein such as a regulation activating other enzymes by sensing cellular concentration of Ca⁺² via calmodulin, a regulation enhancing a blood coagulation rate by activating a serine protease activity through an antihemophilic factor or a regulation of a kinase activity by eliminating an inhibitory subunit of a protein kinase A using cyclic AMP; fourth, a regulation mechanism to activate an enzyme through addition of a phosphoryl group from ATP to the enzyme by a protein kinase and a reversible inactivation modification of a covalent bond by hydrolyzing a phosphoryl group from an enzyme activated by a protein phosphatase; and fifth, a irreversible enzyme activity regulation mechanism using hydrolysis of peptide bonds in zymogens or proenzymes.

Of aforementioned mechanisms, a conversion of proenzymes to their active forms via proteolytic cleavage is an irreversible enzyme activity regulation mechanism which peptide bonds of zymogens or proenzymes are hydrolyzed to convert them to their active forms. The active form of the enzyme by proteolytic cleavage has been known to include its active form such as Lp-PLA₂ (Lipoprotein-associated phospholipase 2), thrombin, urokinase, trypsin, chemotrypsin, elastase and subtilisin.

In binding to a substrate after an enzyme is converted to its active form, two hypotheses are as follows to elucidate a structural conformation change in the active form of the enzyme: first, lock and key hypothesis is a hypothesis that the binding of an enzyme inhibitor to the active form of the enzyme exerts no influence on the structural conformation change in the active form of the enzyme; and second, a induced fit hypothesis is a hypothesis that the binding of an enzyme inhibitor to the active form of the enzyme induces the structural conformation change in the active form of the enzyme.

Lp-PLA₂ is an enzyme activated by proteolytic cleavage of prophosphatase A2 encoded by PLA2G7 gene in human, which is a protein with a molecular weight of 45 kDa consisting of 441 amino acids. In addition, a human mRNA full-length sequence and protein sequence of Lp-PLA₂ correspond to GenBank accession number NM_005084 and NP_005075, respectively. Also, Lp-PLA₂ is PAF (platelet-activating factor), and is known to catalyze degradation of PAF to biologically inactivated products, LYSO-PAF and acetate, via hydrolysis of acetyl group.

It has been known that Lp-PLA₂ is moved with LDL (low-density lipoprotein) in blood and about 20% of is related to HDL (high-density lipoprotein). Lp-PLA₂ is produced by inflammatory cells, and hydrolyzes phospholipids oxidized in LDL. More specifically, Lp-PLA₂ hydrolyzes lipolipids of LDL to lysoPC (lysophosphatidylcholine) and oxNEFA (xidized nonesterified fatty acids), and then oxidized LDL penetrates an arterial endothelial layer and is absorbed into macrophage, resulting in foam cell formation. The foam cells are lysed by lymphocytes and their remnants are gradually enhanced, leading to narrow blood vessel. As a result, the remnants are release into arterial blood vessel to block blood vessel or to induce inflammation.

Conventional Lp-PLA₂ activity assay requires a luminescent or radio-active Lp-PLA₂ substrate and a counter. More practically, Lp-PLA₂ activity assay using a luminescent or fluorescent Lp-PLA₂ substrate needs two washing steps and particular concentration of Lp-PLA₂ for specificity because the substrate is not specific to Lp-PLA₂. In addition, Lp-PLA₂ activity assay using a radio-active Lp-PL-A₂ substrate has the problems such as: (a) addition of BSA (bovine serum albumin) and centrifugation to precipitate a non-cutting or cutting radio-active Lp-PLA₂ substrate; (b) optimization of a chemicals and a buffer using metal ions for substrate precipitation; (c) requirement of a scintillation counter for measuring enzyme activity; and (d) complexity of POCT (Point of Care Testing).

Currently, the method for quantitatively or qualitatively determining the active form of the enzyme has widely utilized an immunological method such as general antigen-antibody reaction.

However, most immunological methods are a method to detect direct binding of a signal-labeled antibody to a target enzyme or its active form, and their demerits are as follows: (a) long time consumable since they utilize complicated steps for analyzing detection signal; and (b) poor reliability in view of result accuracy.

Accordingly, there has been urgently demanded a novel method capable of detecting or analyzing an active form of an enzyme, particularly an active form of an enzyme activated by proteolytic cleavage in a qualitative and quantitative manner.

Throughout this application, various publications and patents are referred and citations are provided in parentheses. The disclosures of these publications and patents in their entities are hereby incorporated by references into this application in order to fully describe this invention and the state of the art to which this invention pertains.

### DETAILED DESCRIPTION OF THE INVENTION

### Technical Purposes of This Invention

The present inventors have made intensive studies to develop a qualitative or quantitative method for analyzing an active form of an enzyme activated by proteolytic cleavage in a rapid manner. As results, we have discovered that the presence or absence of the active form of the enzyme, or the amount thereof was determined in a quick and accurate manner using an enzyme inhibitor and a binding agent specifically bound to the active form of the enzyme.

Accordingly, it is an object of this invention to provide a method for analyzing an active form of an enzyme activated by proteolytic cleavage in a qualitative or quantitative manner.

It is another object of this invention to provide a kit for analyzing an active form of an enzyme activated by proteolytic cleavage in a qualitative or quantitative manner.

Other objects and advantages of the present invention will become apparent from the following detailed description together with the appended claims and drawings.

### Technical Solutions of This Invention

In one aspect of this invention, there is provided a method for analyzing an active form of an enzyme activated by proteolytic cleavage in a qualitative or quantitative manner, comprising:
(a) contacting the active form of the enzyme activated by proteolytic cleavage in a sample of interest to an inhibitor as a capturing agent to the active form of the enzyme;
(b) forming a capturing agent-active form-detecting agent complex by contacting to the resultant of the step (a) a binding agent as a detecting agent capable of binding to the enzyme, the active form of the enzyme or both; and
(c) detecting the capturing agent-active form-detecting agent complex, wherein the detection of the complex indicates the existence of the active form of the enzyme in the sample of interest.

The present inventors have made intensive studies to develop a qualitative or quantitative method for analyzing an active form of an enzyme activated by proteolytic cleavage in a rapid manner. As results, we have discovered that the presence or absence of the active form of the enzyme, or the amount thereof was determined in a quick and accurate manner using an enzyme inhibitor and a binding agent specifically bound to the active form of the enzyme.

The present invention is a method capable of qualitatively or quantitatively analyzing an active form of an enzyme activated by prateolytic cleavage in a specific and rapid manner.

The term "qualitative analysis" used herein in an active form of an enzyme refers to analyze the presence or absence of the active form of the target enzyme in a sample. The term "quantitative analysis" used herein in an active form of an enzyme means analyze the amount of the active form of the target enzyme (for example, concentration) in a sample.

Each step in the present invention may be explained as follows:

### Step (a): contact of the active form of the enzyme and an enzyme inhibitor thereof as a capturing agent

Initially, an enzyme inhibitor for an active form of an enzyme is bound to the active form of the enzyme by contacting the active form of the enzyme in a sample of interest to the enzyme inhibitor as a capturing agent.

The active form of the enzyme capable of qualitatively or quantitatively analyzing in the present invention includes the active form of the enzyme activated by all type of proteolytic cleavages which have been found in naturally-occurring organisms.

The expression "an active form of an enzyme or an active enzyme form" used herein means a form or structure of an enzyme capable of having an enzyme activity in itself by proteolytic cleavage.

In qualitatively or quantitatively analyzing the active form of the enzyme activated by proteolytic cleavage, the active form of the enzyme utilized in the present invention includes preferably Lp-PLA₂ (lipoprotein-associated phospholipase A₂), TAFIa (activated thrombin-activatable fibrinolysis inhibitor), blood coagulation factor V, coagulation factor VII, coagulation factor IX, coagulation factor X, coagulation factor XI, coagulation factor XII, thrombin, trypsin, chemotrypsin, plasmin, u-PA (urokinase-type plasminogen activator), tPA (tissue plasminogen activator), elastase, subtilisin, kallikrein, cathepsin G, collagen, concanavalin A, TPA (12-O-tetradecanoylphorbol-13 acetate) or TGF-β (transforming growth factor-β), more preferably Lp-PLA₂, TAFIa, blood coagulation factor Va, coagulation factor VII, coagulation factor IX, coagulation factor X, coagulation factor XI, coagulation factor XII or thrombin, and much more preferably, Lp-PLA₂ or TAFIa.

According to this invention, an enzyme inhibitor for an active form of an enzyme is utilized as a capturing agent.

The term "enzyme inhibitor" used herein refers to a molecule reducing an enzyme activity through binding to an enzyme. The enzyme inhibitor binds to an active site of an enzyme, thereby blocking an interaction of the enzyme with a substrate and may be bound to the enzyme in a reversible or irreversible manner.

The term "enzyme inhibitor for an active form of an enzyme" used herein means a molecule reducing an enzyme activity via binding to an active form of an enzyme.

An enzyme inhibitor for an active form of an enzyme utilized in the present invention includes an enzyme inhibitor capable of binding to an active form of an enzyme, or to both a proenzyme and an active form of an enzyme. According to a preferable embodiment, the enzyme inhibitor for an active form of an enzyme utilized in the present invention is an enzyme inhibitor specifically binding to only the active form of the enzyme.

A variety of commercially accessible drug molecules include enzyme inhibitors.

An enzyme inhibitor for an active form of an enzyme useful in the present invention includes a natural inhibitor, for example Apo C-III against Lp-PLA₂. In addition, an enzyme inhibitor for an active form of an enzyme useful in the present invention includes a non-natural inhibitor, for example a synthetic inhibitor. In this connection, Darapladib may be useful in the present invention as a synthetic inhibitor to Lp-PLA₂.

Preferably, the inhibitor utilizes in this invention includes proteins (fragment, antibody, domain, etc.), peptides (for example, L- or D-amino acid oligopeptide), DNA aptamer, RNA aptamer, PEA (Peptide Nuclei acids) and organic compounds.

The inhibitor utilized in this invention refers to a substance that may inhibit a function of an active form of an enzyme through modification or non-modification of structure in the active enzyme form via binding to the active form of the enzyme. Preferably, the inhibitor utilized in this invention includes an inhibitor capable of inhibiting a function of an active form of an enzyme without modification of structure in the active enzyme form via binding to the active form of the enzyme.

In the meantime, the inhibitor useful in the present invention includes an inhibitor capable of competitive or uncompetitive binding with a substrate to a substrate binding site in the active form of the enzyme.

According to a preferable embodiment, the inhibitor useful in the present invention includes an inhibitor capable of competitive binding with a substrate to a substrate binding site in the active form of the enzyme.

The capturing agent in step (a) of this invention may be bound to an active form of an enzyme directly or under the condition linked on solid substrate.

According to a preferable embodiment, the capturing agent in step (a) of this invention may specifically bind to an active form of an enzyme under the condition linked on solid substrate

In a capturing agent linked on solid substrate, the solid substrate capable of utilizing in the present invention includes various solid substrates known in the art, for example including a solid substrate consisting of glass, gold, silver, aluminum, chrome, silicon, germanium, gallium arsenide, gargeum, SiN₄, modified silicon nitrocellulose, polyvinyliden fluoride, polystyrene, polytetrafluoroethylene, polycarbonate, nylon or fiber.

To immobilize an active form of an enzyme on the solid substrate useful in the present invention, diverse linker molecules known in the art may be attached on the solid substrate.

As described below, using a two-dimensional format, it is preferable that the capturing agent is immobilized on a plate with flat surface. In a three-dimensional format, the capturing agent is preferable to be immobilized on a bead or particle,

### Step (b): formation of capturing agent-active form of the enzyme-detecting agent complex

The detecting agent contacts a resultant of the step (a), leading to a capturing agent-active form of the enzyme-detecting agent complex.

The detecting agent would exhibit no specificity against an active form of an enzyme, and a binding agent capable of binding to an enzyme, an active enzyme form, or both may be utilized as a detecting agent.

The detecting agent binds to the active form of the enzyme bound to enzyme inhibitor thereof in the step (a).

The detecting agent may be utilized as a form bound to a bead (for example, microbead) or particle (for example, microparticle or nanoparticle). Using the detecting agent linked on a bead or particle, the bead or particle preferably includes a metal bead or particle, for example including magnetic bead, magnetic particle, gold bead or gold particle.

The binding agent useful in the present invention includes a binding agent capable of binding to an enzyme or an active enzyme form. Preferably, the binding agent of this invention includes a binding agent capable of binding to an active enzyme form or both an enzyme and an active enzyme form, and more preferably, a binding agent capable of specifically binding to an active enzyme form.

According to a preferable embodiment, the binding agent useful in the present invention includes an oligopeptide, an antibody (for example, monoclonal antibody, polyclonal antibody or chimeric antibody), a ligand, an oligonucleotide, a PNA (Peptide nucleic acid) or an aptamer (Bock LC et al., Nature 355(6360):564-6(1992); Hoppe-Seyler F, But K, J Mol Med. 78(8):426-30(2000); Cohen BA, Colas P, Brent R., Proc Natl Acad Sci URSA. 95(24):14272-7(1998)), more preferably an antibody, and much more preferably, a monoclonal antibody.

The present invention may be performed not only through the steps consisting of (a) binding of an active enzyme form to a capturing agent; (b) binding of a capturing agent-active enzyme form to a binding agent; and (c) detecting a signal, but also through the steps consisting of (a) simultaneous binding of an active enzyme form to both a capturing agent and a binding agent; and (b) detecting a signal.

According to a preferable embodiment, the present invention may be carried out by simultaneous binding of an active enzyme form to both a capturing agent and a binding agent.

### Step (c): detection of a capturing agent-active enzyme form-detecting agent complex

Finally, a capturing agent-active enzyme form-detecting agent complex is detected. The detection of the complex represents the existence of the active form of the enzyme in the sample of interest.

The detection of the capturing agent-active enzyme form-detecting agent complex may be carried out using a variety of methods. For example, the detection of the complex may be completed using the detecting agent linked with a detectable signal generating label which generates a signal. In addition, the detection of the complex may be carried out by an anti-detecting agent antibody bound to the label capable of generating the detectable signal (for example, secondary antibody in ELISA).

According to a preferable embodiment, the detecting agent includes the label capable of generating the detectable signal, and the step (c) may be performed by detecting the signal generated from the labels linked to the defecting agent.

According to a preferable embodiment, the step (c) is carried out by adding the anti-detecting agent antibody bound to the label capable of generating the detectable signal and then detecting the signal therefrom.

According to a preferable embodiment, a detectable signal generating label linked to a detecting agent or an anti-detecting agent antibody includes chemical labels (*e.g.,* biotin), enzyme labels (*e.g*., alkaline phosphatase, peroxidase, β-galactosidase and β-glucosidase), radio-labels (*e.g*., I¹²³ and C¹⁴), fluorescent labels (*e.g.*, fluorescein, TAMRA, Cy5, Cy3, HEX, TET, Dabsyl and FAM), luminescent labels, chemiluminescent labels, FRET (fluorescence resonance energy transfer) labels or metal labels (*e.g*., gold and silver), more preferably enzyme labels, fluorescent labels or luminescent labels, and most preferably, fluorescent labels.

The conventional methods to detect a target proenzyme or an active enzyme form have utilized a method to detect a signal after direct binding of a signal label to a target proenzyme or an active enzyme form or adding of a signal label-bound antibody. However, these methods require long time consumable and numerous instruments to detect a target proenzyme or an active enzyme form due to complicated procedures (diverse reagents and multiple washing steps).

The present invention is a method capable of specifically detecting an active form of a target enzyme using both an enzyme inhibitor for an active enzyme form and a signal-labeled binding agent, contributing to not only a convenient and quick detection but also outstanding detection accuracy to an active form of a target enzyme.

The step (c) in this invention may be carried out by measuring the signal generated from the label of the detecting agent linked to the active form of the enzyme.

Final analysis in the step (c) of the present invention may be carried out using an automated device well-known in the art such as "reader" or "scanner".

According to a preferable embodiment, the step (c) in this invention may be performed using an optical sensor, an optical detector equipped with a light source and a light detector, an optical detector measuring absorbance or fluorescence, a UV detector, a radiation detector, a confocal microscope detector, a CCD (charge-coupled device) camera or a microplate reader, and more preferably, an optical sensor, an optical detector equipped with a light source and a light detector, an optical detector measuring absorbance or fluorescence or a microplate reader.

According to a preferable embodiment, the present invention further includes a step washing a resultant of the step (b) between the step (b) and the step (c).

Where an alkaline phosphatase is utilized as a label linked to the detecting agent or anti-detecting agent antibody, the substrate may include a chromogenic substrate such as bromochloroindolylphosphate (BCIP), nitro blue tetrazolium (NBT), naphthol-AS-B1-phosphate and ECF (enhanced chemifluorescence), and in a horseradish peroxidase, the substrate may include a substrate such as chloronaphtol, aminoethylcarbazol, diaminobenzidine, D-luciferin, lucigenin (bis-N-methylacridinium nitrate), resorufin benzyl ether, luminol, Amplex Red reagent (10-acetyl-3,7-dihydroxyphenoxazine), HYR (p-phenylenediamine-HCl and pyrocatechol), TMB (tetramethylbenzidine), ABTS (2,2'-Azine-di[3-ethylbenzthiazoline sulfonate]), o-phenylenediamine (OPD), naphtol/pyronine, glucose oxidase and t-NBT (nitroblue tetrazolium), and m-PMS (phenzaine methasulfate). Aforementioned substrate is added to the resultant of the step (b), and the step (c) is carried out by measuring color or luminescence thereafter.

The present invention is a method capable of determining an active form of an enzyme in a sample activated by proteolytic cleavage in a qualitative and/or quantitative manner.

According to a preferable embodiment, the sample containing an active form of an enzyme is derived from mammals, birds, reptiles or amphibians, more preferably mammals, and most preferably, human,

Much more preferably, the sample incudes blood, plasma, serum, saliva, urine, mother's milk, sweat, tissue extract, tumor extract, joint fluid, spinal fluid, seminal fluid or vaginal discharge of mammals, still much more preferably blood, plasma, serum, tissue extract or tumor extract of mammals, and most preferably, blood, plasma or serum of mammals.

The present invention may be utilized for detecting and determining various active enzyme forms associated with a disease or disorder. Without limitation, the present method may be applied to detect an active enzyme form which causes a disease or disorder through conversion of a proenzyme to an active form of an enzyme.

According to a preferable embodiment, the active enzyme form of interest in this invention includes an active enzyme form associated with arteriosclerosis, more preferably atherosclerosis, arteriolosclerosis, mediasclerosis, angina pectoris, myocardial infarction, cerebrovascular dementia, transient ischemic attack of brain, ischemic stroke, hemorrhage, stroke, cerebral thrombosis, cerebral embolism or peripheral arterial obstructive disease.

Meanwhile, the above-mentioned method of the present invention may be carried out using two kinds of format: (i) a two-dimensional format; and (ii) a three-dimensional format.

Most difference between a two-dimensional format and a three-dimensional format is a form of a capturing agent. In the two-dimensional format, a capturing agent is bound on a solid substrate such as a plate, whereas a capturing agent is lined to a bead or particle in the three-dimensional format.

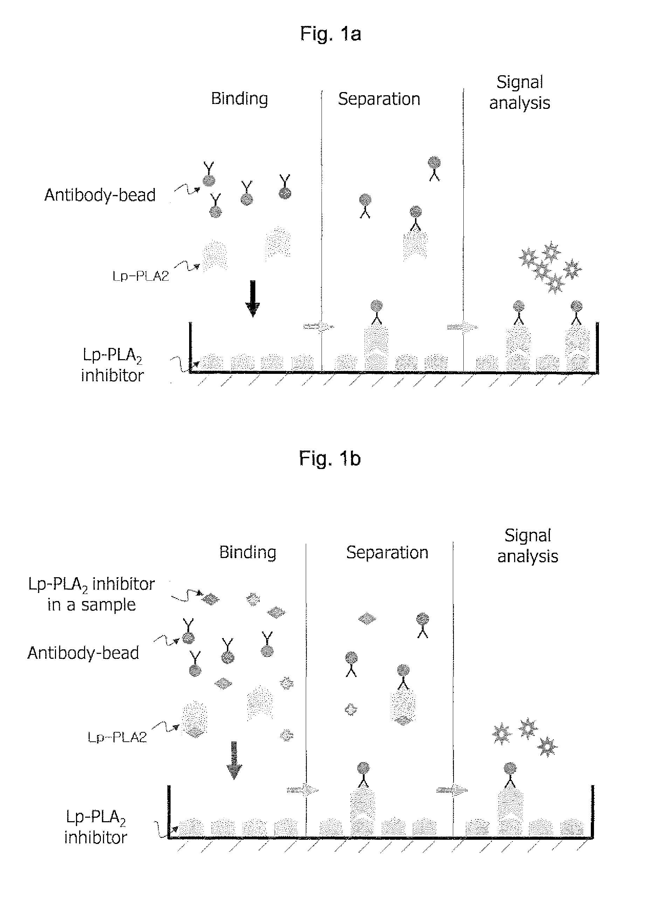

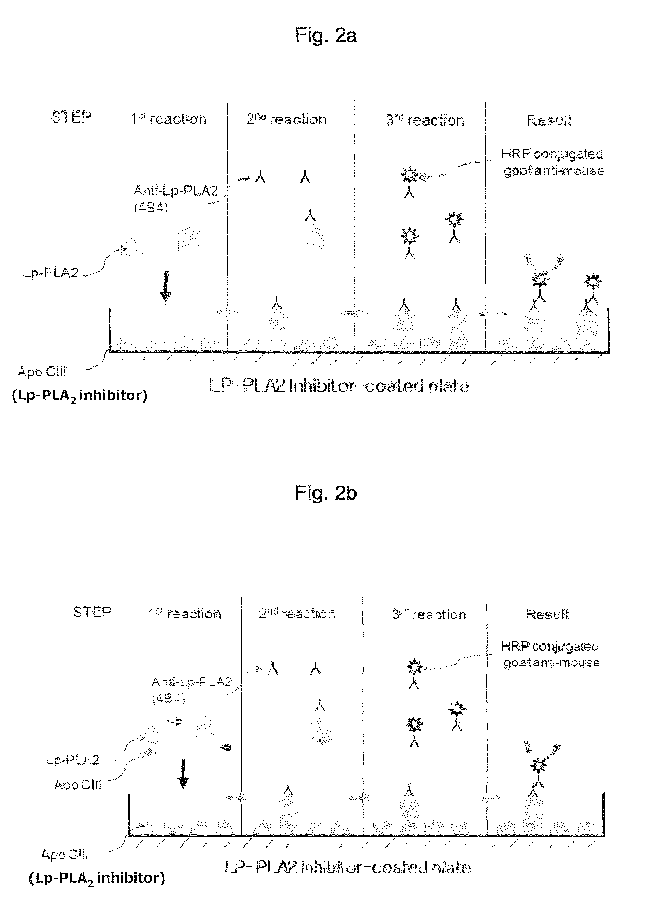

The two-dimensional format is described above in detail, and shown in Fig. 1a and Fig. 2b.

The three-dimensional format utilizes a capturing agent bound to a bead or particle. For example, where Apo C-III to be an active Lp-PLA₂ inhibitor is utilized as a capturing agent, and an antibody bound to an active Lp-PLA₂ as a detecting agent, a magnetic particle-conjugated Apo C-III and a label-baund Lp-PLA₂ antibody are simultaneously reacted with a sample. After termination, the resultant is subjected to a magnetic field, leading to separation of Apo C-III-magnetic particle-active Lp-PLA₂ form-Lp-PLA₂ antibody complex. Subsequently, the signal from the label is detected to analyze an active Lp-PLA₂ form in a sample in a qualitative and/or quantitative manner.

In another aspect of this invention, there is provided a method for analyzing an active form of an enzyme activated by proteolytic cleavage in a qualitative or quantitative manner, comprising:
(a) contacting the active form of the enzyme activated by proteolytic cleavage in a sample of interest to a binding agent as a capturing agent capable of binding to the enzyme, the active form of the enzyme or both;
(b) forming a capturing agent-active form-detecting agent complex by contacting to the resultant of the step (a) an inhibitor as a detecting agent capable of binding to the active form of the enzyme; and
(c) detecting the capturing agent-active form-detecting agent complex, wherein the detection of the complex indicates the existence of the active form of the enzyme in the sample of interest.

Since the second aspect of the present invention is the same to the first aspect described above except that each binding agent and detecting agent is utilized as a capturing agent and an enzyme inhibitor, the common descriptions between them are omitted in order to avoid undue redundancy leading to the complexity of this specification.

In still another aspect of this invention, there is provided a kit for analyzing an active form of an enzyme activated by proteolytic cleavage in a qualitative or quantitative manner, comprising: (a) an inhibitor to the active form of the enzyme activated by proteolytic cleavage as a capturing agent; and (b) a binding agent capable of binding to the enzyme, the active form of the enzyme or both.

In further still another aspect of this invention, there is provided a kit for analyzing an active form of an enzyme activated by proteolytic cleavage in a qualitative or quantitative manner, comprising: (a) a binding agent bound to the enzyme, the active form of the enzyme or both; and (b) an inhibitor capable of binding to the active form of the enzyme as a detecting agent.

Since the present kit invention embodies the method of this invention, the common descriptions between them are omitted in order to avoid undue redundancy leading to the complexity of this specification.

According to a preferable embodiment, the detecting agent includes a label capable of generating a detectable signal.

According to a preferable embodiment, the kit further includes an anti-detecting agent antibody bound to the label capable of generating the detectable signal.

According to a preferable embodiment, the inhibitor for the active form of the enzyme inhibits a binding of a substrate by competitive binding to a substrate binding site of the active form of the enzyme.

According to a preferable embodiment, the capturing agent is bound to a solid substrate.

According to a preferable embodiment, the binding agent is an antibody.

According to a preferable embodiment, the the active form of the enzyme activated by proteolytic cleavage includes Lp-PLA₂ (lipoprotein-associated phospholipase A₂), TAFIa (activated thrombin-activatable fibrinolysis inhibitor), blood coagulation factor V, coagulation factor VII, coagulation factor IX, coagulation factor X, coagulation factor XI, coagulation factor XII, thrombin, trypsin, chemotrypsin, plasmin, u-PA (urokinase-type plasminogen activator), tPA (tissue plasminogen activator), elastase, subtilisin, kallikrein, cathepsin G, collagen, concanavalin A, TPA (12-O-tetradecanoylphorbol-13 acetate) or TGF-β (transforming growth factor-β), more preferably Lp-PLA₂, TAFIa, blood coagulation factor Va, coagulation factor VII, coagulation factor IX, coagulation factor X, coagulation factor XI, coagulation factor XII or thrombin, and most preferably, Lp-PLA₂ or TAFIa,

According to a preferable embodiment, the inhibitor for the active form of the enzyme inhibits a binding of a substrate by competitive binding to a substrate binding site of the active form of the enzyme.

According to a preferable embodiment, the inhibitor for the active form of the enzyme specifically binds to only the active form of the enzyme.

### Effects of This Invention

The features and advantages of the present invention will be summarized as follows:
(a) The present invention relates to a method to qualitatively and quantitatively analyze an active form of an enzyme activated by proteolytic cleavage.
(b) The present invention has a merit of analyzing an active enzyme form in a sample of interest using an active enzyme inhibitor and a binding agent in much rapid and accurate manner compared with a method to determine an active enzyme form using a simple binding agent (*e.g*., antibody).
(c) The present invention may analyze an active enzyme form in a sample of interest using an uncomplicated instrument in a convenient and feasible manner compared with a method to determine an active enzyme form using a simple binding agent.
(d) The present invention may determine a target enzyme or an active enzyme form by simultaneously using an inhibitor and a binding agent in a quick and specific manner, thereby screening drug in a high-throughput manner.
(e) In addition, the present invention may be applied to POCT (Point of care testing) for efficacy and dose of drug to a subject (for example, animal or human) in a clinical monitoring.

### BRIEF DESCRIPTION OF THE DRAWINGS

Figs. 1a-1b schematically represent a procedure to analyze Lp-PLA₂ in a sample in a qualitative and quantitative manner. Fig. 1a schematically represents a procedure to analyze Lp-PLA₂ in a sample in a qualitative and quantitative manner using a Lp-PLA₂ inhibitor immobilized on a solid substrate and and a signal label-conjugated Lp-PLA₂ binding agent (antibody). Fig. 1b schematically shows a procedure to analyze Lp-PLA₂ in a sample in a qualitative and quantitative manner using a Lp-PLA₂ inhibitor immobilized on a solid substrate and a signal label-conjugated Lp-PLA₂ binding agent (antibody), and where Lp-PLA₂ inhibitor is present in a sample, the signal is not detected since Lp-PLA₂ is bound to Lp-PLA₂ inhibitor, thereby no binding to the Lp-PLA₂ inhibitor immobilized on a solid substrate. Therefore, the present method may analyze both the presence of active Lp-PLA₂ in a sample and the amount of active Lp-PLA₂.
Figs 2a-2b represent a diagram about a procedure described in Example 2.

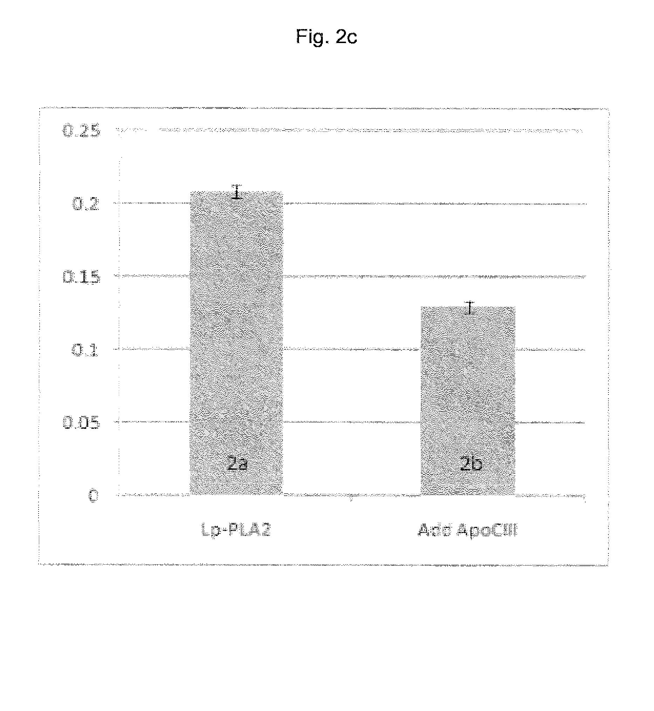

Fig. 2c is a graph showing the results of Example 2. Axis Y indicates absorbance (Ab 620 nm).

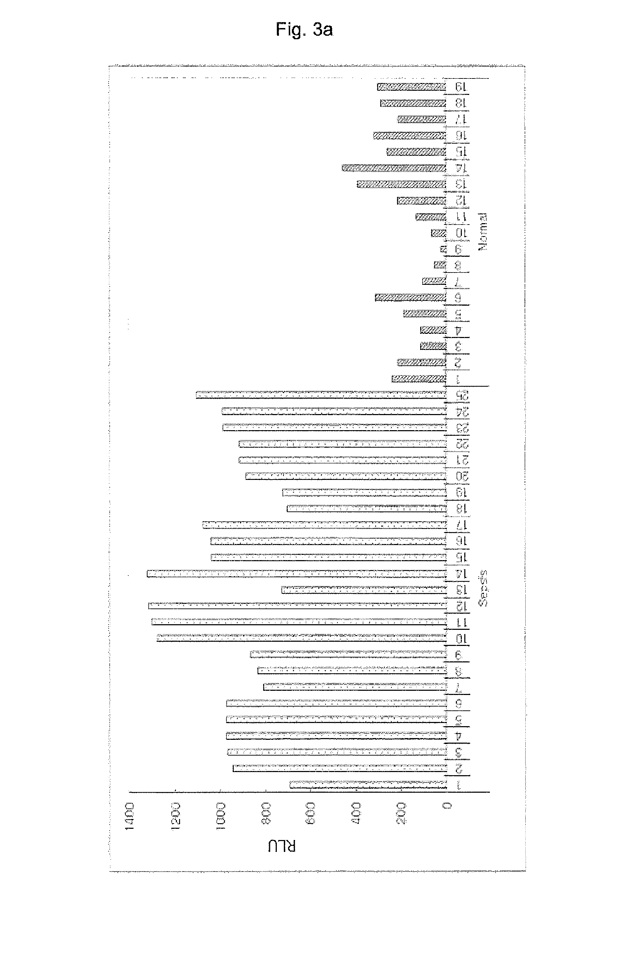

Fig. 3a shows a result to analyze the amount of TAFIa and TAFIai in plasma of healthy subjects and sepsis patients according to the present method. Hatched bars indicate ELISA to measure the amount of TAFIa and TAFIai in plasma of a healthy subject and number 1-25 bars indicate ELISA to measure the amount of TAFIa and TAFIai in plasma of sepsis patients.

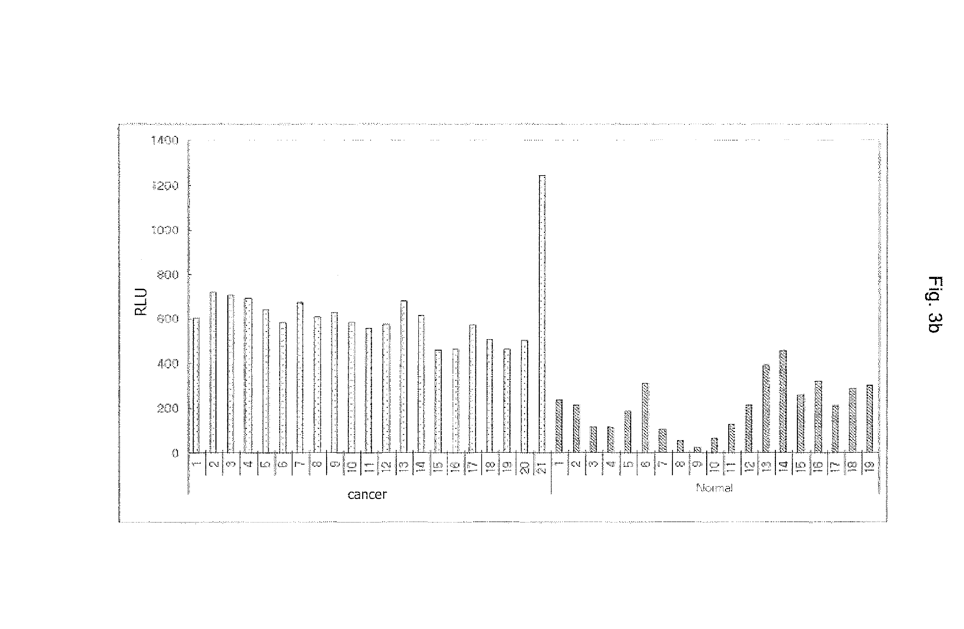

Fig. 3b represents a result to analyze the amount of TAFIa and TAFIai in plasma of healthy subjects and cancer patients according to the present method. Hatched bars indicate ELISA to measure the amount of TAFIa and TAFIai in plasma of a healthy subject and dot bars indicates ELISA to measure the amount of TAFIa and TAFIai in plasma of cancer patients.

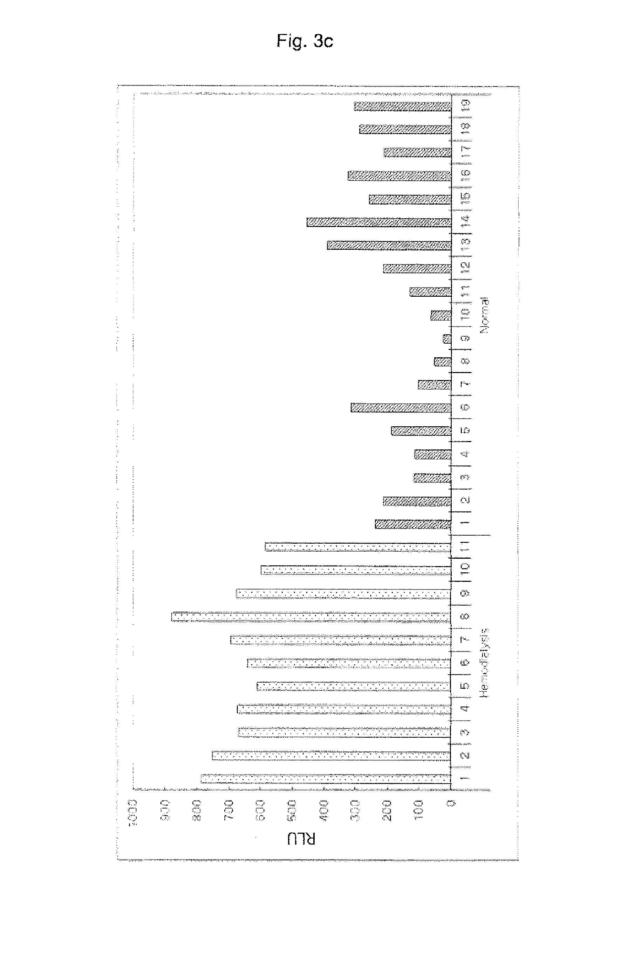

Fig. 3c represents a result to analyze the amount of TAFIa and TAFlai in plasma of healthy subjects and hemodialysis patients according to the present method. Hatched bars indicate ELISA to measure the amount of TAFIa and TAFIai in plasma of a healthy subject and dot bars indicate ELISA to measure the amount of TAFIa and TAFIai in plasma of hemodialysis patients.

### EXAMPLES OF THE INVENTION

The present invention will now be described in further detail by examples. It would be obvious to those skilled in the art that these examples are intended to be more concretely illustrative and the scope of the present invention as set forth in the appended claims is not limited to or by the examples.

### EXAMPLES

Unless otherwise described in the specification, solid/solid, solid/liquid and liquid/liquid indicate (weight/weight) parts by weight or %, (weight/volume) parts by weight or %, and (volume/volume) parts by weight or %, respectively.

### EXAMPLE 1: Analysts of an Active Form of LPL (lipoprotein lipase) Using ain Apo C-III (Apolipoprotein C-III)-Coated Chip Materials and Instruments

Materials and instruments utilized in this experiment are as follows:
Apo C-III (Sigma, USA), LPL antibody (mouse anti-lipoprotein lipase monoclonal antibody, Unconjugated; Clonetech: Abcam, USA), Avidin (Sigma, USA), EDC (Ethyl-3-(3-dimethylaminopropyl)carbodiimide hydrochloride; Thermo, USA), NHS (N-hydroxysulfosuccinimide sodium salt; Fluka, USA), Sulfo-NHS-biotin (EZ-Link Suiro-NHS-LC-LC-Biotin; PIERCE, USA), Fluorescent particle (FluorSpheres carboxylate-modified microspheres, 0,2 µm, dark red fluorescent (660/680), Molecular Probes), BSA(Bovine serum albumin; BOVOGEN, Australia), Dextran (Dextran from Leuconostoc sp., Fluka, USA), Tween-20 (Sigma, USA), Dialysis menbran (Spectra/pro dialysis membrane 12,000-14,000, Spectrum Lab, USA), Centrifuge (MICRO 17RT, Hanil Science, Republic of Korea), FREND reader (Nanoentech, Inc., Republic of Korea), UV Spectrophotometer (UV-1800, SHIMADZU, Japan), Incubator (HANBAEK, Republic of Korea)

### Apo C-II biotinylation

Apo C-III was diluted at a concentration of 1 mg/ml in PBS (phosphate buffered saline), and 10 mM biotin was prepared using distilled water. Afterwards, 1 ml Apo C-III (1 mg/ml) was mixed with 27 µl biotin (10 mM) and the mixture was incubated at room temperature with agitation. The solution was added to a dialysis membrane and then dialyzed in PBS. And then, the buffer was exchanged with 1 Liter PBS every 3 hrs (total 3 times). The absorbance in the dialyzed reactant was measured at 280 nm and the reactant was stored at 4°C.

### Fluorescent anti-LPL antibody conjugates

Anti-LPL antibody (1 mg/ml) was prepared with 50 mM MES buffer (pH 6.2). Each EDC and NHS was added to the anti-LPL antibody solution (1 mg/ml) corresponding to a concentration of 50 mM and 100 mM. The mixture was immediately mixed with 0.2 weight/volume% fluorescent particle and incubated with agitation at room temperature for 3 hrs. The reactant solution was centrifuged at 12,000 g for 15 min and then the supernatant was removed. The precipitates was resuspended in PBS and centrifuged at 12,000 g for 15 min, followed by removing the supernatant. Afterwards, the precipitates were resuspended in PBS supplemented with 1% BSA, and the concentration of fluorescent particle was adjusted to 0.2 weight/volume%. Above-mentioned anti-LPL antibody fluorescence conjugates were stored at 4°C.

### Chip preparation

After avidin was diluted in PBS solution at a concentration of 100 µg/ml, each EDC (50 mM) and NHS (100 mM) was added. The prepared avidin solution (1 µl) was dotted on a PMMA (poly(methyl methacrylate) slide induced with carboxyl group. The slide was incubated at 37°C incubator for 1.5 hrs. After the slide was washed with 0.1 M Tris solution (pH 7.4), biotinylated APO C-III (1.5 µl) diluted at 10 µg/ml was dotted at the slide portion dotted with avidin solution. APO C-III was diluted with PBS solution supplemented with 1% BSA. The biotinylated APO C-III-dotted slide was incubated at 37°C incubator for 1.5 hrs. Afterwards, the slide was washed with PBS solution containing 1% BSA, 0.05% Tween 20 and 0.5% dextran, and then dried at room temperature. Anti-LPL antibody fluorescence conjugates (1.2 µl) was dotted in the slide and dried, which was adjusted at a concentration of 0.2 weight/volume% using 0.1 M Tris solution (pH 7.4) supplemented with 1% BSA, 0.01% Tween20 and 3% dextran. Other slide capable of inducing and controlling the flow of dried slide was conjugated, resulting in a testable chip.

### Method

Sample (30 µl; serum or plasma of patient with or without lipoprotein lipase) was dropped to an inlet part of the prepared chip. After 5 min, the testable chip was inserted into a FREND reader (Nanoentech, Inc.). The presence of LPL in sample was quantitatively indicated in the display screen of FREND reader after about 40 seconds, and this quantitative display refers to a degree of LPL activation.

### EXAMPLE 2: Analysis of an Active Form of Lp-PLA₂ (lipoprotein associated phospholipase 2) Using an Apo C-III (Apolipoprotein C-III)-coated microplate

### Materials and Instruments

Materials and instruments utilized in this experiment are as follows;
Apo C-III (A3106, Sigma, USA), Lp-PLA₂ antibody (mouse anti-lipoprotein lipase monoclonal antibody, Clonetech 4B4, diaDexus, USA), Lp-PLA₂ (diaDexus, USA), Horse radish peroxidase conjugated Goat anti-mose IgG (Sigma, USA), Microplate (NUNC, Denmark), BSA (Bovine serum albumin; BOVOGEN, Australia), Tween-20 (Sigma, USA), Tetramehtylbenzidine (TMB) (Sigma, USA), Incubator (HANBAEK, Republic of Korea), Infinit M200 (TECAN, Swiss)

### Apo C-III coating

After Apo C-III was diluted with 100 mM carbonate buffer (pH 9.5) at a concentration of 2 µg/ml, the Apo C-III solution (100 µl) was added to each well in a microplate, which was coated at 37°C incubator for 2 hrs. And then, the Apo C-III solution was removed from each well and 200 µl PBS supplemented with 1% BSA was added to each well. After incubation at 37°C incubator, the solution was removed from each well that was then dried.

### Method

Tween-20 (0.05%)-containing PBS buffer (pH 7.4) was used for dilution, reaction and washing buffer in the experiment. Each 100 µl Lp-PLA₂ (1 µg/ml) was added to Apo C-III-coated microplate well (Fig. 2a). Or, Lp-PLA₂ and Apo C-III was diluted at a final concentration of 1 µg/ml and 0.5 µg/ml, respectively, and the solution (100 µl) was added to Apo C-III-coated microplate well (Fig. 2b). Each reaction preparation was incubated at 37°C incubator for 50 min, and then removed from the well, followed by washing 2 times, Anti-Lp-PLA₂ antibody solution (100 µl) diluted to 5 µg/ml was added to the washed well and incubated at 37°C Incubator for 50 min. The solution was removed and each well was washed 2 times. HRP-conjugated goat anti-mouse antibody solution (100 µl; 1:5,000 dilution) was added to each washed well and incubated at 37°C incubator for 30 min. The solution was removed and each well was washed 3 times. TMB solution (100 µl) was added to each washed well and incubated at room temperature for 30 min. After 100 µl H₂SO₄ (2 N) was added to each washed well, the absorbance was measured. The measurement of absorbance utilized TECAN Infinit M200: wavelength measurement, 450 nm; and calibration wavelength, 620 nm.

### Result

Apo C-III is coated on a microplate and has an inhibitory activity for Lp-PLA₂. In the present system, Apo C-III is responsible for a capturing agent against Lp-PLA₂. Where both Lp-PLA₂ and free Apo C-III are added to a sample, Lp-PLA₂ may be not bound to Apo C-III coated on a microplate because it binds to free Apo C-III. In this connection, free Apo C-III and Apo C-III coated on a microplate competitively binds to Lp-PLA₂.

**Table 1.**

| | Final signal intensity (absorbance: Ab 620nm) | |
|---|---|---|
| | Lp-PLA₂ alone | Lp-PLA₂ and Apo C-III |
| Mean | 0.208 | 0.130 |
| Standard deviation | 0,004 | 0.004 |

As shown in Fig. 2c and Table 1, in a sample containing both Lp-PLA₂ and free Apo C-III, competition binding response was developed by immobilized Apo C-III as a capturing agent, thereby 37.6% reduction of signal compared with in a sample under the condition without free Apo C-III. In this connection, these results suggest that PLP activity may be assessed using certain inhibitor against Apo C-III.

### EXAMPLE 3: TAFIa and TAFIai Analysis Using a PTCI-coated microplate

### Materials and method

To measure TAFI and TAFIai acitivity in plasma derived from patients with sepsis, cancer and hemodialysis, HTS (High-Throughput Screening) analysis according to the present invention was carried out.

First, PTCI (potato tuber carboxypeptidase inhibitor; Sigma) was mixed with sodium carbonate-bicarbonate buffer, thereby adjusting to a concentration of 2 µg/ml. The mixture (100 µl) was added to a 96-well plate (Nunc Modules for Fluorescence) and then, the plate was kept to stand at 4°C overnight. The plate was washed 3 times with PBS (Phosphate buffered saline, pH 7.4; Sigma). After Blockace (snow brand milk products Co. Ltd., Japan) was mixed with distilled water at a concentration of 10 µg/ml, the mixture (150 µl) was added to each well and incubated in a moisture chamber at room temperature for 4 hrs, followed by washing with PBS 3 times..

Each plasma was extracted from patients with sepsis, cancer and hemodialysis, and diluted up to 30 volute% by mixing TBST (Tris buffered saline, with Tween-20, pH 8.0; Sigma). The plasma-containing solutions (100 µl) were seeded on a prerequisite PTCI-coated plate and incubated at 37°C for 1 hr, followed by washing 3 times with TBST.

Subsequently, each anti-TAFI antibody (Monoclonal mouse anti-TAFI, Hytest, 4TA1_16C5) and Blockace was diluted with TBST to a concentration of 1 µg/ml and 0.8 µg/ml, and the diluent (100 µl) was divided into a well, respectively. The plate containing a dilution solution was incubated at 37°C for 1 hr and washed with TBST 3 times. Next, after anti-IgG (Anti-Mouse IgG-Peroxidase antibody, sigma, A8924) HRP (horseradish peroxidase) was diluted with TBST at a dilution ratio of 1 x 10⁴, and the concentration of Blockace was subjected to 0.8 µg/ml, they (100 µl) were divided into each well. The well was incubated at 37°C for 1 hr and washed with TEST 3 times. Finally, ECL solution (100 µl; Thermo, 37070) was added to each well, and the signal intensity was measured using a Victor Plate reader (VICTOR³, PerkinFimer).

### Results

### TAFIa-PTCI and TAFIai-PTCI response In a Patient with sepsis

Using blood sample of a sepsis patient, the amount of TAFIa and TAFIai in plasma was assessed. After plasma in a healthy subject and sepsis patient was added to a PTCI-coated 96 multi-well plate, the concentration of TAFIa and TAFIai was measured using a HRP-conjugated secondary antibody. As a result, it could be appreciated that the amount of TAFIa and TAFIai as a TAFI isomer in the plasma of the systemic sepsis patient was enhanced much more remarkable than that in the healthy subject (Fig. 3a).

### TAFIa-PTCI and TAFIai-PTCI response in a Patient with cancer

Using blood sample as described above in the sepsis patient, the amount of TAFIa and TAFIai in plasma was analyzed in a cancer patient. As same as the sepsis patient, the amount of TAFIa and TAFIai in the plasma of the cancer patient was enhanced much more noticeable than that in the healthy subject (Fig. 3b).

### TAFIa -PTCI and TAFIai-PTCI response in a Patient with hemodialysys

Using blood sample as described above, the amount of TAFIa and TAFIai in plasma was measured in a hemodialysis patient. As same as the sepsis patient, the amount of TAFIa and TAFIai in the plasma of the hemodialysis patient was enhanced much more significant than that in the healthy subject (Fig. 3c).

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. A method for analyzing an active form of an enzyme activated by proteolytic cleavage in a qualitative or quantitative manner, comprising:
(a) contacting the active form of the enzyme activated by proteolytic cleavage in a sample of interest to an inhibitor as a capturing agent to the active form of the enzyme;
(b) forming a capturing agent-active form-detecting agent complex by contacting to the resultant of the step (a) a binding agent as a detecting agent capable of binding to the enzyme, the active form of the enzyme or both; and
(c) detecting the capturing agent-active form-detecting agent complex, wherein the detection of the complex indicates the existence of the active form of the enzyme in the sample of interest.

2. A method for analyzing an active form of an enzyme activated by proteolytic cleavage in a qualitative or quantitative manner, comprising:
(a) contacting the active form of the enzyme activated by proteolytic cleavage in a sample of interest to a binding agent as a capturing agent capable of binding to the enzyme, the active form of the enzyme or both;
(b) forming a capturing agent-active form-detecting agent complex by contacting to the resultant of the step (a) an inhibitor as a detecting agent capable of binding to the active form of the enzyme; and
(c) detecting the capturing agent-active form-detecting agent complex, wherein the detection of the complex indicates the existence of the active form of the enzyme in the sample of interest.

3. The method according to claim 1 or claim 2, wherein the detecting agent comprises a label capable of generating a detectable signal and the step (c) is carried out by detecting the signal from the detecting agent-bound label.

4. The method according to claim 1 or claim 2, wherein the step (c) is carried out by incubating with an anti-detecting agent antibody having a label capable of generating a detectable signal.

5. The method according to claim 1 or claim 2, wherein the active form of the enzyme activated by proteolytic cleavage comprises Lp-PLA₂ (lipoprotein-associated phospholipase A₂), TAFIa (activated thrombin-activatable fibrinolysis inhibitor), blood coagulation factor V, coagulation factor VII, coagulation factor IX, coagulation factor X, coagulation factor XI, coagulation factor XII, thrombin, trypsin, chemotrypsin, plasmin, u-PA (urokinase-type plasminogen activator), tPA (tissue plasminogen activator), elastase, subtilisin, kallikrein, cathepsin G, collagen, concanavalin A, TPA (12-O-tetradecanoylphorbol-13 acetate) or TGP-β (transforming growth factor-β).

6. The method according to claim 5, wherein the active form of the enzyme comprises Lp-PLA₂, TAFIa, blood coagulation factor Va, coagulation factor VII, coagulation factor IX, coagulation factor X, coagulation factor XI, coagulation factor XII or thrombin.

7. The method according to claim 6, wherein the active form of the enzyme comprises Lp-PLA₂ or TAFIa.

8. The method according to dam or claim 2, wherein the inhibitor to the active form of the enzyme inhibits the binding of a substrate to a substrate binding site of the active form of the enzyme in a competitive binding manner.

9. The method according to claim 1 or claim 2, wherein the inhibitor to the active form of the enzyme binds specifically to the active form of the enzyme.

10. The method according to claim 1 or claim 2, wherein the capturing agent is bound to a solid substrate.

11. The method according to claim 1 or claim 2, wherein the binding agent comprises an oligopeptide, an antibody, a ligand, an oligonucleotide, PNA (Peptide nucleic acid) or an aptamer.

12. The method according to claim 11, wherein the binding agent is the antibody.

13. The method according to claim 1 or claim 2, wherein the step (a) and the step (b) are carried out in a simultaneous manner.

14. The method according to claim 1 or claim 2, wherein the sample comprises blood, plasma, serum, saliva, urine, mother's milk, sweat, tissue extract, tumor extract, joint fluid, spinal fluid, seminal fluid or vaginal discharge.

15. The method according to claim 14, wherein the sample comprises blood, plasma, serum, tissue extract or tumor extract.

16. The method according to claim 1 or claim 2, wherein the active form of the enzyme comprises an active form of an enzyme associated with arteriosclerosis.

17. A kit for analyzing an active form of an enzyme activated by proteolytic cleavage in a qualitative or quantitative manner, comprising:
(a) an inhibitor to the active form of the enzyme activated by proteolytic cleavage as a capturing agent; and
(b) a binding agent capable of binding to the enzyme, the active form of the enzyme or both.

18. A kit for analyzing an active form of an enzyme activated by proteolytic cleavage in a qualitative or quantitative manner, comprising:
(a) a binding agent bound to the enzyme, the active form of the enzyme or both; and
(b) an inhibitor capable of binding to the active form of the enzyme as a detecting agent,

19. The kit according to claim 17 or claim 18, wherein the detecting agent comprises a label capable of generating a detectable signal.

20. The kit according to claim 17 or claim 18, wherein the kit further comprises an anti-detecting agent antibody having a label capable of generating a detectable signal.

21. The kit according to claim 17 or claim 18, wherein the inhibitor to the active form of the enzyme inhibits the binding of a substrate to a substrate binding site of the active form of the enzyme in a competitive binding manner.

22. The kit according to claim 17 or claim 18, wherein the capturing agent is bound to a solid substrate.

23. The kit according to claim 17 or claim 18, wherein the binding agent is an antibody.

24. The kit according to claim 17 or claim 18, wherein the active form of the enzyme activated by proteolytic cleavage comprises Lp-PLA₂ (lipoprotein-associated phospholipase A₂), TAFIa (activated thrombin-activatable fibrinolysis inhibitor), blood coagulation factor V, coagulation factor VII, coagulation factor IX, coagulation factor X, coagulation factor XI, coagulation factor XII, thrombin, trypsin, chemotrypsin, plasmin, u-PA (urokinase-type plasminogen activator), tPA (tissue plasminogen activator), elastase, subtilisin, kallikrein, cathepsin G, collagen, concanavalin A, TPA (12-O-tetradecanoylphorbol-13 acetate) or TGF-β (transforming growth factor-β).

25. The kit according to claim 24, wherein the active form of the enzyme comprises Lp-PLA₂, TAFIa, blood coagulation factor Va, coagulation factor VII, coagulation factor IX, coagulation factor X, coagulation factor XI, coagulation factor XII or thrombin.

26. The kit according to claim 25, wherein the active form of the enzyme comprises Lp-PLA₂ or TAFIa.

27. The kit according to claim 17 or claim 18, wherein the inhibitor to the active form of the enzyme inhibits the binding of a substrate to a substrate binding site of the active form of the enzyme in a competitive binding manner.

28. The kit according to claim 17 or claim 18, wherein the inhibitor to the active form of the enzyme binds specifically to the active form of the enzyme.
